(19) Europäisches Patentamt
European Patent Office
Office européen des brevets

(11) Veröffentlichungsnummer: **0 151 429**
**A2**

# (12) EUROPÄISCHE PATENTANMELDUNG

(21) Anmeldenummer: 85100638.7

(22) Anmeldetag: 23.01.85

(51) Int. Cl.$^4$: **C 07 C 69/712**
C 07 D 213/30, A 01 N 43/40
A 01 N 37/40, C 07 C 67/14
C 07 C 67/31, C 07 C 79/35
C 07 C 143/68, C 07 C 139/0-

(30) Priorität: 04.02.84 DE 3403974

(43) Veröffentlichungstag der Anmeldung:
14.08.85 Patentblatt 85/33

(84) Benannte Vertragsstaaten:
AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Förster, Heinz, Dr.
Am Eckbusch 47
D-5600 Wuppertal 1(DE)

(72) Erfinder: Priesnitz, Uwe, Dr.
Severinstrasse 58
D-5650 Solingen 1(DE)

(72) Erfinder: Riebel, Hans-Jochem, Dr.
In der Beek 92
D-5600 Wuppertal 1(DE)

(72) Erfinder: Eue, Ludwig, Dr.
Paul-Klee-Strasse 36
D-5090 Leverkusen 1(DE)

(72) Erfinder: Schmidt, Robert R., Dr.
Im Waldwinkel 110
D-5060 Bergisch-Gladbach(DE)

## (54) Optisch active Phenoxypropionsäure-Derivate.

(57) Neue R-Enantiomere von Phenoxypropionsäure-Derivaten der Formel

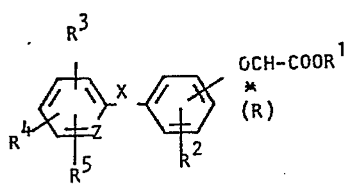

(I)

in welcher

Z für Stickstoff oder die Gruppierung –CR steht, worin

R für Wasserstoff oder Halogen steht,

X für Sauerstoff oder Schwefel steht,

$R^1$ für Alkyl, gegebenenfalls substituiertes Cycloalkyl, gegebenenfalls substituiertes Cycloalkenyl, gegebenenfalls substituiertes Bicycloalkyl oder gegebenenfalls substituiertes Bicycloalkenyl mit jeweils mindestens einem asymmetrisch substituierten Kohlenstoffatom steht,

$R^2$ für Wasserstoff, Cyano oder Nitro steht,

$R^3$ und $R^4$ gleich oder verschieden sind und für Wasserstoff oder Halogen stehen und

$R^5$ für Wasserstoff, Halogen oder Trihalogenmethyl steht,

mehrere Verfahren zur Herstellung der neuen Stoffe und deren Verwendung also Herbizide.

EP 0 151 429 A2

Croydon Printing Company Ltd.

0151429

BAYER AKTIENGESELLSCHAFT 5090 Leverkusen, Bayerwerk

Konzernverwaltung RP
Patentabteilung

Dü/Ke

I b

# Optisch aktive Phenoxypropionsäure-Derivate

Die vorliegende Erfindung betrifft neue R-Enantiomere*) von Phenoxypropionsäure-Derivaten, mehrere Verfahren zu deren Herstellung und ihre Verwendung als Herbizide.

Es ist bereits bekannt, daß zahlreiche Diphenylether-Derivate herbizide Eigenschaften besitzen (vgl. DE-OS 22 23 894). So kann zum Beispiel das Racemat des 2-[4-(2,4-Dichlor-phenoxy)-phenoxy]-propionsäure-methylesters zur Bekämpfung von Unkraut eingesetzt werden. Die Wirkung dieses Stoffes ist gut, jedoch werden bei geringen Aufwandmengen einige Unkräuter nicht immer voll erfaßt.

---

*) Unter R-Enantiomeren sind im vorliegenden Fall jeweils diejenigen optisch aktiven Verbindungen zu verstehen, welche am asymmetrisch substituierten Kohlenstoffatom in der Seitenkette die R-Konfiguration aufweisen.

Es wurden nun neue R-Enantiomere von Phenoxypropionsäure-Derivaten der Formel (I),

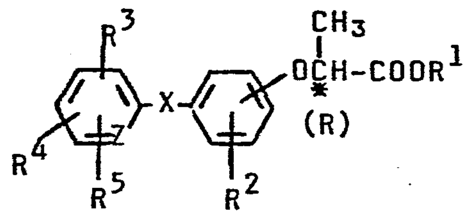

(I)

in welcher

Z für Stickstoff oder die Gruppierung -CR steht, worin R für Wasserstoff oder Halogen steht,

X für Sauerstoff oder Schwefel steht,

$R^1$ für Alkyl, gegebenenfalls substituiertes Cycloalkyl, gegebenenfalls substituiertes Cycloalkenyl, gegebenenfalls substituiertes Bicycloalkyl oder gegebenenfalls substituiertes Bicycloalkenyl mit jeweils mindestens einem asymmetrisch substituierten Kohlenstoffatom steht,

$R^2$ für Wasserstoff, Cyano oder Nitro steht,

$R^3$ und $R^4$ gleich oder verschieden sind und für Wasserstoff oder Halogen stehen und

$R^5$ für Wasserstoff, Halogen oder Trihalogenmethyl steht,

gefunden.

Weiterhin wurde gefunden, daß man die neuen R-Enantiomeren von Phenoxypropionsäure-Derivaten der Formel (I) erhält, wenn man

(a) R-Enantiomere von Phenoxypropionsäurechloriden der Formel (II),

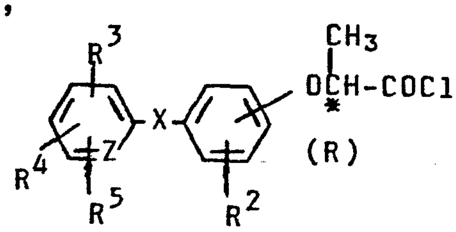

(II)

in welcher

$R^2$, $R^3$, $R^4$, $R^5$, Z und X die oben angegebenen Bedeutungen haben,

mit Verbindungen der Formel (III),

$R^1OH$ (III)

in welcher

$R^1$ die oben angegebene Bedeutung hat,

gegebenenfalls in Gegenwart eines Säureakzeptors sowie gegebenenfalls in Gegenwart eines Verdünnungsmittels umsetzt, oder

(b) Phenole der Formel (IV),

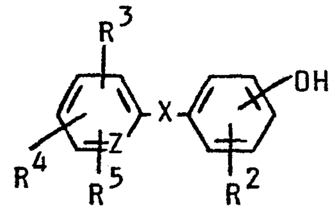

(IV)

in welcher

$R^2$, $R^3$, $R^4$, $R^5$, Z und X die oben angegebenen Bedeutungen haben,

mit S-Enantiomeren von Propionsäure-Derivate der Formel (V),

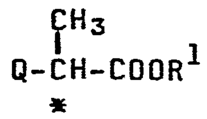

(S)

(V)

in welcher

$R^1$ die oben angegebene Bedeutung hat und

Q für Tosylat oder Mesylat steht,

gegebenenfalls in Gegenwart eines Säureakzeptors sowie gegebenenfalls in Gegenwart eines Verdünnungsmittels umsetzt,

oder

(c) R-Enantiomere von Phenoxypropionsäure-Derivaten der Formel (Ia),

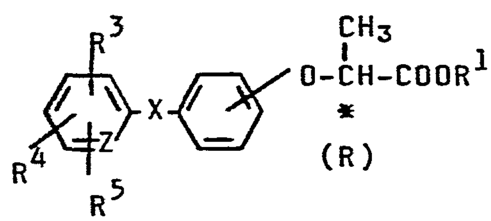

(Ia)

in welcher

$R^1$,$R^3$,$R^4$,$R^5$, Z und X die oben angegebenen Bedeutungen haben,

mit Kupfernitrat in Gegenwart von Essigsäureanhydrid und Eisessig umsetzt.

Schließlich wurde gefunden, daß sich die neuen R-Enantiomeren von Phenoxypropionsäure-Derivaten der Formel (I) durch eine hervorragende herbizide Wirksamkeit auszeichnen.

Ueberraschenderweise besitzen die erfindungsgemäßen R-Enantiomeren von Phenoxypropionsäure-Derivaten der Formel (I) wesentlich bessere herbizide Eigenschaften als der aus dem Stand der Technik bekannte 2-[4-(2,4-Dichlorphenoxy)-phenoxy]-propionsäuremethylester, welcher ein hoch wirksamer Wirkstoff gleicher Wirkungsart ist. Vor allem lassen sich mit Hilfe der erfindungsgemäßen Wirkstoffe einige Ungräser, die von dem 2-[4-(2,4-Dichlorphenoxy)-phenoxy]-propionsäuremethylester nicht voll erfaßt werden, wirksam bekämpfen.

Die erfindungsgemäßen R-Enantiomeren von Phenoxypropionsäure-Derivaten sind durch die Formel (I) allgemein definiert.

In dieser Formel, in der das asymmetrisch substituierte Kohlenstoffatom durch ein (*) gekennzeichnet ist, stehen vorzugsweise

Z für Stickstoff oder die Gruppierung -CR, worin R vorzugsweise für Wasserstoff, Chlor oder Brom steht,

X für Sauerstoff oder Schwefel,

$R^1$ für Alkyl mit 4 bis 12 Kohlenstoffatomen mit mindestens 1 asymmetrisch substituierten Kohlenstoffatom sowie für (1R,2S)-Fenchyl; (1S,2R)-Bornyl; (1R,2R)-iso-Bornyl; (1R)-Myrtenyl; (1S,2S,4S)-Dihydrocarvyl; (1R,3R,4S)-Menthyl und (1S,3S,4R)-Menthyl,

$R^2$ für Wasserstoff, Cyano oder Nitro,

$R^3$ für Wasserstoff, Chlor oder Brom,

$R^4$ für Wasserstoff, Chlor oder Brom und

$R^5$ für Wasserstoff, Fluor, Chlor, Brom, Trifluormethyl, Difluorchlormethyl oder Trichlormethyl.

Eine besonders bevorzugte Gruppe von erfindungsgemäßen Verbindungen sind diejenigen Stoffe der Formel (I), in denen

Z für Stickstoff steht,

X für Sauerstoff steht,

$R^1$ für Alkyl mit 4 bis 8 Kohlenstoffatomen und mindestens 1 asymmetrischen Kohlenstoffatom steht,

$R^2$ für Wasserstoff oder Nitro steht,

$R^3$ für Wasserstoff steht,

$R^4$ für Wasserstoff oder Chlor steht und

$R^5$ für Chlor oder Trifluormethyl steht.

Eine andere Gruppe von besonders bevorzugten erfindungsgemäßen Verbindungen sind diejenigen Stoffe der Formel (I), in denen

Z für Stickstoff steht,

X für Sauerstoff steht,

$R^1$ für (1R,2S)-Fenchyl; (1S,2R)-Bornyl; (1R,2R)-iso-Bornyl; (1R)-Myrtenyl; (1S,2S,4S)-Dihydrocarvyl; (1R,3R,4S)-Menthyl und (1S,3S,4R)-Menthyl steht,

$R^2$ für Wasserstoff oder Nitro steht,

$R^3$ für Wasserstoff steht,

$R^4$ für Wasserstoff oder Chlor steht und

$R^5$ für Chlor oder Trifluormethyl steht.

Eine weitere Gruppe von besonders bevorzugten erfindungsgemäßen Verbindungen sind diejenigen Stoffe der Formel (I), in denen

Z für die Gruppierung -CR steht, worin
R für Wasserstoff oder Chlor steht,

X für Sauerstoff steht,

$R^1$ für Alkyl mit 4 bis 8 Kohlenstoffatomen und mindestens 1 asymmetrischen Kohlenstoffatom steht,

$R^2$ für Wasserstoff oder Nitro steht,

$R^3$ für Wasserstoff oder Chlor steht,

$R^4$ für Wasserstoff oder Chlor steht und

$R^5$ für Chlor oder Trifluormethyl steht.

Eine andere Gruppe von besonders bevorzugten erfindungsgemäßen Verbindungen sind schließlich diejenigen Stoffe der Formel (I), in denen

Z für die Gruppierung -CR steht, worin R für Wasserstoff oder Chlor steht,

X für Sauerstoff steht,

$R^1$ für (1R,2S)-Fenchyl; (1S,2R)-Bornyl; (1R,2R)-iso-Bornyl; (1R)-Myrtenyl; (1S,2S,4S)-Dihydrocarvyl; (1R,3R,4S)-Menthyl und (1S,3S,4R)-Menthyl steht,

$R^2$ für Wasserstoff oder Nitro steht,

$R^3$ für Wasserstoff oder Chlor steht,

$R^4$ für Wasserstoff oder Chlor steht und

$R^5$ für Chlor oder Trifluormethyl steht.

Verwendet man das R-Enantiomere des 2-[4-(3,5-Dichlor-pyridyl-2-oxy)-phenoxy]-propionsäurechlorids und (2S)-2-Methylbutan-1-ol als Ausgangsstoffe, so kann der Verlauf des erfindungsgemäßen Verfahrens (a) durch das folgende Formelschema wiedergegeben werden :

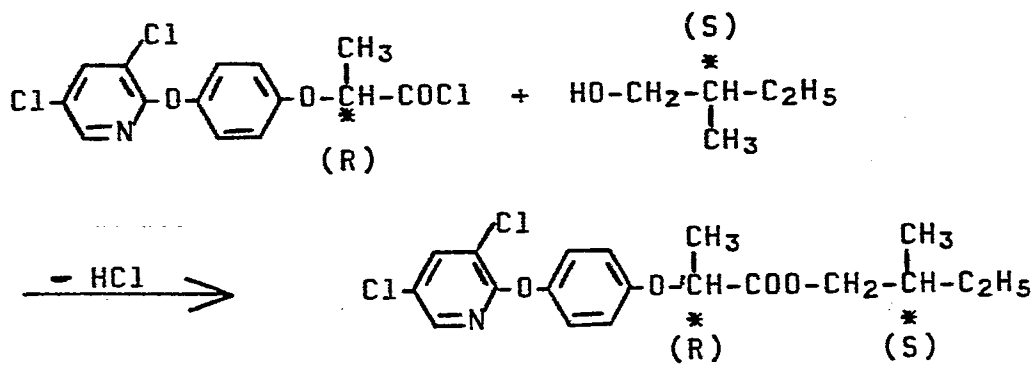

Verwendet man 3-(2,6-Dichlor-4-trifluormethyl-phenoxy)-phenol und 2-Tosyloxy-(2S)-propionsäure-(1S,3S,4R)-menth-3-ylester als Ausgangsstoffe, so kann der Verlauf des erfindungsgemäßen Verfahrens (b) durch das folgende Formelschema wiedergegeben werden :

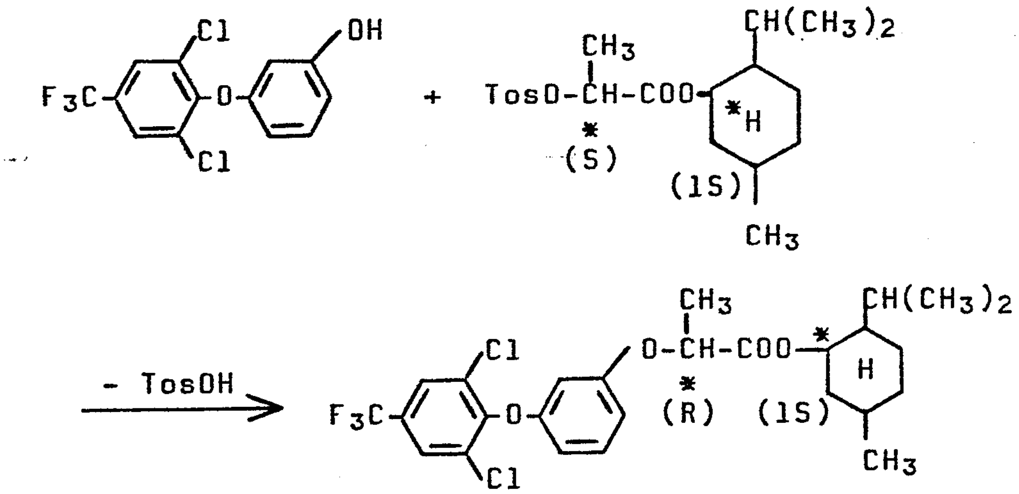


Tos = 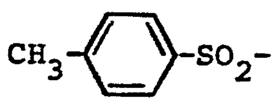

Verwendet man (2R)-2-[3-(2,6-Dichlor-4-trifluormethyl-phenoxy)-phenoxy]-propionsäure-(1S,3S,4R)-menth-3-ylester als Ausgangsstoff, Kupfernitrat als Reaktionskomponente und Essigsäureanhydrid und Eisessig als Verdünnungsmittel, so kann der Verlauf des erfindungsgemäßen Verfahrens (c) durch das folgende Formelschema wiedergegeben werden :

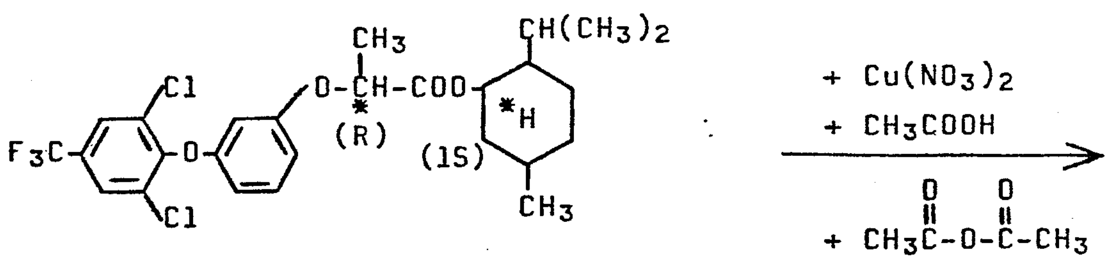

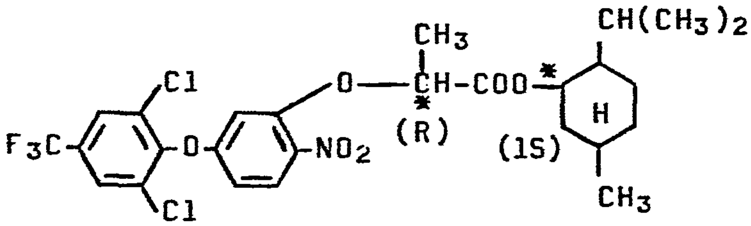

Die beim erfindungsgemäßen Verfahren (a) als Ausgangsstoffe benötigten R-Enantiomeren von Phenoxypropionsäurechloriden sind durch die Formel (II) definiert. In dieser Formel haben

X, Z, $R^2$,$R^3$,$R^4$ und $R^5$ vorzugsweise diejenigen Bedeutungen, die bereits im Zusammenhang mit der Beschreibung der erfindungsgemäßen Stoffe der Formel (I) vorzugsweise für diese Reste genannt wurden.

Als Beispiele für die Verbindungen der Formel (II) seien genannt : die R-Enantiomeren von 2-[3-(2,6-Dichlor-4-trifluormethyl-phenoxy)-phenoxy]-, 2-[3-(2,6-Dichlor-4-trifluormethyl-phenoxy)-6-nitro-phenoxy]-, 2-[3-(2-Chlor-4-trifluormethyl-phenoxy)-phenoxy]-, 2-[3-(2-Chlor-4-trifluormethyl-phenoxy)-6-nitro-phenoxy]-, 2-[4-(2,4-Dichlor-phenoxy)-phenoxy]-, 2-[4-(4-Chlor-2-trifluormethyl-phenoxy)-phenoxy]-, 2-[4-(4-Trifluormethyl-phenoxy)-phenoxy]-, 2-[4-(3,5-Dichlor-pyridyl-2-oxy)-phenoxy]-, 2-[4-(5-Trifluormethyl-pyridyl-2-oxy)-phenoxy]- und 2-[4-(3-Chlor-5-trifluormethyl-pyridyl-2-oxy)-phenoxy]-propionsäurechlorid; 2-[3-(2,6-Dichlor-4-trifluormethyl-phenylthio)-phenoxy]-, 2-[3-(2,6-Dichlor-4-trifluormethyl-phenylthio)-6-nitro-phenoxy]-, 2-[3-(2-Chlor-4-trifluormethyl-phenylthio)-phenoxy]-, 2-[3-(2-Chlor-4-trifluormethyl-phenylthio)-6-nitro-phenoxy]-,

2-[4-(2,4-Dichlor-phenylthio)-phenoxy]-, 2-[4-(4-Chlor-2-trifluormethyl-phenylthio)-phenoxy]-, 2-[4-(4-Trifluormethyl-phenylthio)-phenoxy]-, 2-[4-(3,5-Dichlor-pyridyl-2-thio)-phenoxy]-, 2-[4-(5-Trifluormethyl-pyridyl-2-thio)-phenoxy]- und 2-[4-(3-Chlor-5-trifluormethyl-pyridyl-2-thio)-phenoxy]-propionsäurechlorid.

Die R-Enantiomeren der Phenoxypropionsäurechloride der Formel (II) sind bekannt oder lassen sich nach im Prinzip bekannten Methoden herstellen (vgl. EP-OS 0 002 800).

Die beim erfindungsgemäßen Verfahren (a) außerdem als Ausgangsstoffe benötigten Verbindungen sind durch die Formel (III) definiert. In dieser Formel hat $R^1$ vorzugsweise die Bedeutung, die bereits im Zusammenhang mit der Beschreibung der erfindungsgemäßen Stoffe der Formel (I) vorzugsweise für diesen Rest genannt wurde.

Als Beispiele für die Verbindungen der Formel (III) seien die in der folgenden Tabelle 1 aufgeführten Stoffe genannt :

$$R^1OH \qquad (III)$$

Tabelle 1 :

| $R^1OH$ | | $R^1OH$ |
|---|---|---|
| 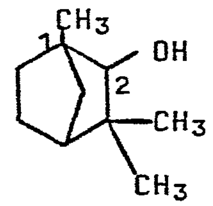 | (1R,2S)-Fenchol | 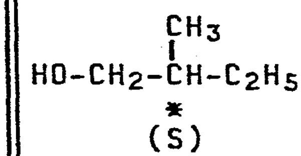 |
| 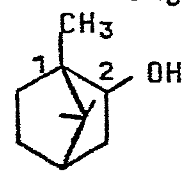 | (1S,2R)-Borneol<br>(1R,2R)-iso-Borneol | 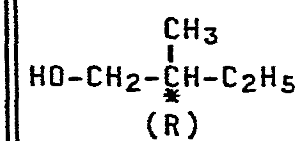 |
| 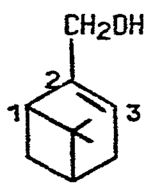 | (1R)-Myrtenol | |
| 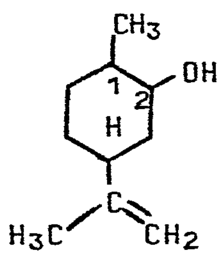 | (1S,2S,4S)-Dihydrocarveol | |
| 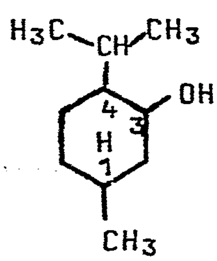 | (1R,3R,4S)-Menthol<br>(1S,3S,4R)-Menthol | |

Die Verbindungen der Formel (III) sind allgemein bekannte Verbindungen der organischen Chemie.

Das erfindungsgemäße Verfahren (a) kann gegebenenfalls in Gegenwart von Säureakzeptoren durchgeführt werden. Als Säureakzeptoren können alle üblicherweise für derartige Umsetzungen verwendbaren Säurebindemittel eingesetzt werden. Vorzugsweise in Frage kommen : Alkalihydroxide, Erdalkalihydroxide und -oxide, wie z.B. Natrium- und Kaliumhydroxid, Calciumhydroxid und Calciumoxid, Alkalicarbonate und -alkoholate, wie Natrium- und Kaliumcarbonat, Natrium- und Kaliummethylat bzw. -ethylat, ferner aliphatische, aromatische und heterocyclische Amine, beispielsweise Triethylamin, Trimethylamin, Dimethylanilin, Dimethylbenzylamin, 1,5-Diazabicyclo[4,3,0]non-5-en (DBN), 1,8-Diazabicyclo[5,4,0]undec-7-en (DBU) und Pyridin.

Das erfindungsgemäße Verfahren (a) kann gegebenenfalls in Gegenwart von Verdünnungsmitteln durchgeführt werden. Als Verdünnungsmittel kommen bei der Durchführung des erfindungsgemäßen Verfahrens (a) alle inerten organischen Solventien in Betracht. Vorzugsweise verwendbar sind aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol; Ether, wie Diethyl- und Dibutylether, Glycoldimethylether und Diglycoldimethylether, Tetrahydrofuran und Dioxan; Ketone, wie Aceton, Methyl-ethyl-, Methyl-isopropyl- und Methyl-isobutyl-keton; Ester, wie Essigsäure-methylester und -ethylester; Nitrile, wie z.B. Acetonitril und Propionitril;

Amide, wie z.B. Dimethylformamid, Dimethylacetamid und N-Methyl-pyrrolidon, sowie Dimethylsulfoxid, Tetramethylensulfon und Hexamethylphosphorsäuretriamid.

Die Reaktionstemperaturen können bei der Umsetzung nach dem erfindungsgemäßen Verfahren (a) innerhalb eines größeren Bereiches variiert werden. Im allgemeinen arbeitet man bei Temperaturen zwischen -20°C und +160°C, vorzugsweise zwischen 0°C und +140°C.

Das erfindungsgemäße Verfahren (a) wird im allgemeinen bei Normaldruck durchgeführt. Es ist jedoch auch möglich, unter erhöhtem oder vermindertem Druck zu arbeiten.

Zur Durchführung des erfindungsgemäßen Verfahrens (a) werden die Ausgangsstoffe der Formeln (II) und (III) im allgemeinen in angenähert äquimolaren Mengen eingesetzt. Es ist jedoch auch möglich, eine der beiden jeweils eingesetzten Komponenten in einem größeren Ueberschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt.

Das erfindungsgemäße Verfahren (a) kann jedoch auch ohne Säureakzeptoren und ohne Verdünnungsmittel durchgeführt werden.

Die Aufarbeitung erfolgt jeweils nach üblichen Verfahren. Im allgemeinen geht man so vor, daß man das Reaktionsgemisch mit Wasser versetzt, mehrfach mit einem mit Wasser

wenig mischbaren Lösungsmittel extrahiert, die vereinigten organischen Phasen nacheinander mit verdünnter wässriger Alkalilauge und Wasser wäscht, dann trocknet und durch Abziehen des Lösungsmittels unter vermindertem Druck einengt.

Die beim erfindungsgemäßen Verfahren (b) als Ausgangsstoffe benötigten Verbindungen sind durch die Formel (IV) definiert. In dieser Formel haben X, Z, $R^2$,$R^3$,$R^4$ und $R^5$ vorzugsweise die Bedeutungen, die bereits im Zusammenhang mit der Beschreibung der erfindungsgemäßen Verbindungen der Formel (I) vorzugsweise für diese Reste genannt wurden.

Als Beispiele für die Verbindungen der Formel (IV) seien genannt : 3-(2,6-Dichlor-4-trifluormethyl-phenoxy)-, 3-(2,6-Dichlor-4-trifluormethyl-phenoxy)-6-nitro-, 3-(2-Chlor-4-trifluormethyl-phenoxy)-, 3-(2-Chlor-4-trifluormethyl-phenoxy)-6-nitro-, 4-(2,4-Dichlor-phenoxy)-, 4-(4-Chlor-2-trifluormethyl-phenoxy)-, 4-(4-Trifluormethyl-phenoxy)-, 4-(3,5-Dichlor-pyridyl-2-oxy)-, 4-(5-Trifluormethyl-pyridyl-2-oxy)- und 4-(3-Chlor-5-trifluormethyl-pyridyl-2-oxy)-phenol;

3-(2,6-Dichlor-4-trifluormethyl-phenylthio)-, 3-(2,6-Dichlor-4-trifluormethyl-phenylthio)-6-nitro-, 3-(2-Chlor-4-trifluormethyl-phenylthio)-, 3-(2-Chlor-4-trifluormethyl-phenylthio)-6-nitro-, 4-(2,4-Dichlor-phenylthio)-, 4-(4-Chlor-2-trifluormethyl-phenylthio)-, 4-(4-Trifluormethyl-phenylthio)-, 4-(3,5-Dichlor-pyridyl-2-thio)-, 4-(5-Trifluormethyl-pyridyl-2-thio)- und 4-(3-Chlor-5-trifluormethyl-pyridyl-2-thio)-phenol.

Die Verbindungen der Formel (IV) sind bekannt.

Die beim erfindungsgemäßen Verfahren (b) außerdem als Ausgangsstoffe benötigten S-Enantiomeren von Propionsäure-Derivaten sind durch die Formel (V) definiert. In dieser Formel hat $R^1$ vorzugsweise die Bedeutung, die bereits im Zusammenhang mit der Beschreibung der erfindungsgemäßen Verbindungen der Formel (I) vorzugsweise für diesen Rest genannt wurde.

In dieser Formel steht Q für Tosylat ($-O-SO_2-$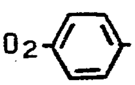$-CH_3$) oder Mesylat ($-O-SO_2-CH_3$).

Die S-Enantiomeren der Propionsäure-Derivate der Formel (V) sind neu. Sie lassen sich nach üblichen Verfahren in einfacher Weise herstellen. So erhält man die S-Enantiomeren der Propionsäure-Derivate der Formel (V), indem man die S-Enantiomeren der Propionylchloride der Formel (VI),

$$Q-\underset{(S)}{\overset{\overset{CH_3}{|}}{\underset{*}{C}}}H-CO-Cl \qquad (VI)$$

in welcher

Q die oben angegebene Bedeutung hat,

mit Verbindungen der Formel (III),

$$R^1OH \qquad (III)$$

in welcher

$R^1$ die oben angegebene Bedeutung hat,

gegebenenfalls in Gegenwart eines Säureakzeptors sowie gegebenenfalls in Gegenwart eines Verdünnungsmittels umsetzt.

Die bei der Herstellung der S-Enantiomeren der Propionsäure-Derivate der Formel (V) als Ausgangsstoffe benötigten S-Enantiomeren von Propionylchloriden der Formel (VI) sind bekannt oder lassen sich nach bekannten Methoden herstellen.

Bei diesem Verfahren zur Herstellung der S-Enantiomeren der Propionsäure-Derivate der Formel (V) entsprechen die Umsetzungsbedingungen denen des erfindungsgemäßen Verfahrens (b).

Der Einsatz von S-Enantiomeren der Propionsäure-Derivate der Formel (V) ist bei dem erfindungsgemäßen Verfahren (b) deshalb erforderlich, weil im Verlauf der Reaktion eine Walden'sche Umkehr am asymmetrisch substituierten Kohlenstoffatom der Propionsäure-Einheit erfolgt.

Bei der Durchführung des erfindungsgemäßen Verfahrens (b) wird im allgemeinen in Gegenwart eines Verdünnungsmittels sowie in Gegenwart eines Säurebindemittels gearbeitet. Hierbei kommen als Verdünnungsmittel und Säureakzeptoren vorzugsweise diejenigen Stoffe in Betracht, die in diesem Zusammenhang bereits im Falle des erfindungsgemäßen Verfahrens (a) als bevorzugt genannt wurden.

Die Reaktionstemperaturen können auch bei dem erfindungsgemäßen Verfahren (b) innerhalb eines größeren Bereiches variiert werden. Im allgemeinen arbeitet man bei Temperaturen zwischen -20°C und +160°C, vorzugsweise zwischen 0°C und +140°C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die Ausgangsstoffe der Formeln (IV) und (V) im allgemeinen in angenähert äquimolaren Mengen eingesetzt. Es ist jedoch auch möglich, eine der beiden Komponenten in einem größeren Ueberschuß zu verwenden. Die Aufarbeitung erfolgt nach üblichen Methoden.

Die beim erfindungsgemäßen Verfahren (c) als Ausgangsstoffe benötigten Verbindungen sind durch die Formel (Ia) definiert. In dieser Formel haben X, Z, $R^1$,$R^3$,$R^4$ und $R^5$ vorzugsweise die Bedeutungen, die bereits im Zusammenhang mit der Beschreibung der erfindungsgemäßen Verbindungen der Formel (I) vorzugsweise für diese Reste genannt wurden.

Die Verbindungen der Formel (Ia) sind erfindungsgemäße Verbindungen. Sie lassen sich in der oben beschriebenen Weise nach Verfahren (a) oder (b) herstellen.

Die Nitrierung wird bei dem erfindungsgemäßen Verfahren (c) vorzugsweise mit Kupfernitrat-Trihydrat durchgeführt. Als Verdünnungsmittel dient dabei ein Gemisch aus Acetanhydrid und Eisessig. Das Verhältnis von Acetanhydrid zu Eisessig kann innerhalb eines bestimmten Bereiches variiert werden. Im allgemeinen setzt man auf 1 Gewichtsteil Acetanhydrid 0,2 bis 3 Gewichtsteile, vorzugsweise 0,4 bis 2 Gewichtsteile Eisessig ein.

Die Reaktionstemperaturen können auch bei dem erfindungsgemäßen Verfahren (c) innerhalb eines größeren Bereiches variiert werden. Im allgemeinen arbeitet man bei Temperaturen zwischen -20°C und +100°C, vorzugsweise zwischen 0°C und +50°C.

Das erfindungsgemäße Verfahren (c) wird im allgemeinen unter Normaldruck durchgeführt. Es ist jedoch auch möglich, unter erhöhtem oder vermindertem Druck zu arbeiten.

Bei der Durchführung des erfindungsgemäßen Verfahrens (c) setzt man auf 1 Mol einer Verbindung der Formel (Ia) im allgemeinen 0,5 bis 1 Mol, vorzugsweise 0,6 bis 0,8 Mol an Kupfernitrat-Trihydrat ein. Im einzelnen verfährt man entweder so, daß man die jeweilige Verbindung der Formel (Ia) in Essigsäureanhydrid gelöst vorlegt und ein Gemisch von Kupfernitrat in Eisessig zutropft, oder so, daß man ein Gemisch aus einer Verbindung der Formel (Ia), Essigsäureanhydrid und Kupfernitrat vorlegt und Eisessig zutropft. Nach beendeter Zugabe wird das Reaktionsgemisch 10 bis 15 Stunden gerührt. Die anschließende Aufarbeitung erfolgt nach üblichen Methoden.

Die neuen Verbindungen fallen in Form von Oelen an, die sich zum Teil nicht unzersetzt destillieren lassen, jedoch durch sogenanntes 'Andestillieren', d.h. durch längeres Erhitzen unter vermindertem Druck auf mäßig erhöhte Temperaturen von den letzten flüchtigen Anteilen befreit und auf diese Weise gereinigt werden können. Zu ihrer Charakterisierung dient der Brechungsindex.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe können vorzugsweise zur Gräserbekämpfung in Zuckerrüben, Sojabohnen und Baumwolle eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. Ammoniumsalze und natürliche Gesteinsmehle, wie Kaoline, Tonerden, Talkum, Kreide, Quarz, Attapulgit,

Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

Herstellungsbeispiele :

Beispiel 1 :

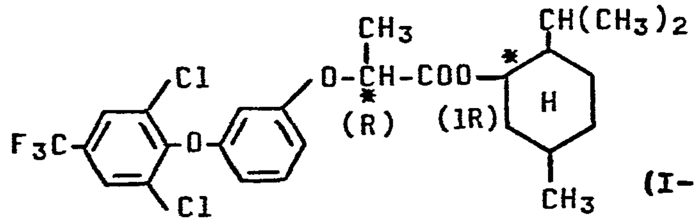

(I-1)

(Verfahren (a))

15,6 g (0,1 Mol) (1R,3R,4S)-Menthol und 42,9 g (0,1 Mol) des R-Enantiomeren von 2-[3-(2,6-Dichlor-4-trifluormethyl-phenoxy)-phenoxy]-propionsäurechlorid wurden unter Rühren so lange auf 100°C bis 120°C erhitzt, bis kein Chlorwasserstoff mehr gebildet wurde (ca. 4 Stunden).

Man erhielt 47,6 g (93 % der Theorie) (2R)-2-[3-(2,6-Dichlor-4-trifluormethyl-phenoxy)-phenoxy]-propionsäure-(1R,3R,4S)-menth-3-ylester.

Brechungsindex : $n_D^{22} = 1,5151$

Drehwert : $[\alpha]_D^{24} = -14,6°$

(1-molare Lösung in Chloroform; Küvettenlänge 10 cm)

Die Herstellung der Verbindung (I-1) in Gegenwart von einem Säureakzeptor und einem Verdünnungsmittel:

Eine Lösung aus 15,6 g (0,1 Mol) (1R,3R,4S)-Menthol und 42,9 g (0,1 Mol) des R-Enantiomeren von 2-[3-(2,6-Dichlor-4-trifluormethyl-phenoxy)-phenoxy]-propionsäurechlorid in 100 ml Toluol wurde bei 20°C unter Rühren mit 8,25 g (0,11 Mol) Pyridin versetzt. Das Gemisch wurde zuerst 5 Stunden bei 20°C und anschließend 5 Stunden bei 60°C gerührt. Das Gemisch wurde dann zweimal mit jweils 100 ml Wasser extrahiert, und die organische Phase wurde eingeengt.

Man erhielt 45 g (88 % der Theorie) der obengenannten Verbindung (I-1).

Beispiel 2 :

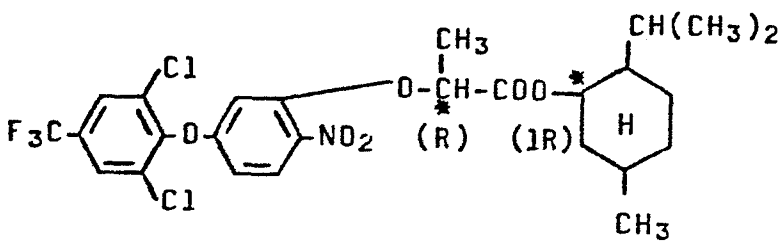

(I-2)

(Verfahren (c))

Zu 38,9 g (0,07 Mol) der Verbindung (I-1) in 25 g (0,25 Mol) Essigsäureanhydrid wurden 10 g (0,042 Mol) Kupfernitrat-Trihydrat gelöst in 42 ml Eisessig in der Weise unter Rühren zugetropft, daß eine Temperatur von 28°C bis 35°C im Reaktionsgemisch gehalten wurde. Anschließend wurde 12 Stunden bei 20°C nachgerührt. Zur Aufarbeitung wurde das Gemisch in 200 ml Wasser eingegossen und zweimal mit jeweils 100 ml Methylenchlorid extrahiert.

Die vereinigten Methylenchloridextrakte wurden über Natriumsulfat getrocknet und anschließend eingeengt. Der Rückstand wurde andestilliert.

Man erhielt 32 g (90 % der Theorie) (2R)-2-[3-(2,6-Dichlor-4-trifluormethyl-phenoxy)-6-nitro-phenoxy]-propionsäure-(1R,3R,4S)-menth-3-ylester.

Brechungsindex: $n_D^{22} = 1,5223$

Drehwert: $[\alpha]_D^{24} = 0°$

(1-molare Lösung in Chloroform; Küvettenlänge 10 cm)

Nach den in den Beispielen 1 und 2 angegebenen Methoden wurden auch die in den folgenden Beispielen aufgeführten Stoffe hergestellt.

Beispiel 3 :

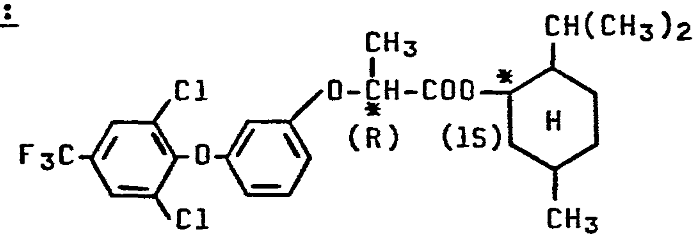

(I-3)

Ausbeute : 90 % der Theorie

$n_D^{22}$ = 1,5196

Drehwert : $[\alpha]_D^{24}$ = +18,7°

(1-molare Lösung in Chloroform; Küvettenlänge 10 cm)

Beispiel 4 :

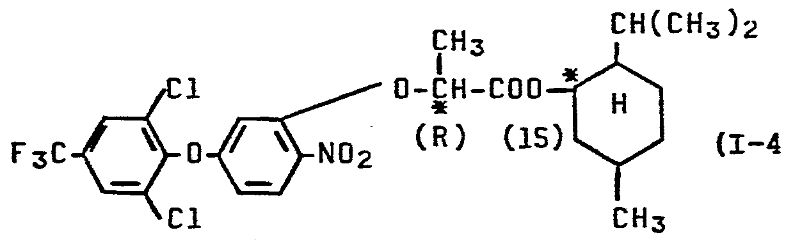

(I-4)

Ausbeute : 87 % der Theorie

$n_D^{22}$ = 1,5248

Drehwert : $[\alpha]_D^{24}$ =-33,2°

(1-molare Lösung in Chloroform; Küvettenlänge 10 cm)

0151429

Beispiel 5 :

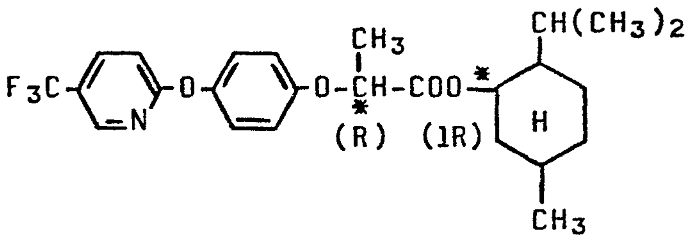

(I-5)

Ausbeute : 86,5 % der Theorie

$n_D^{22}$ = 1,5009

Drehwert : $[\alpha]_D^{24}$ = -0,2°

(1-molare Lösung in Chloroform; Küvettenlänge 10 cm)

Beispiel 6 :

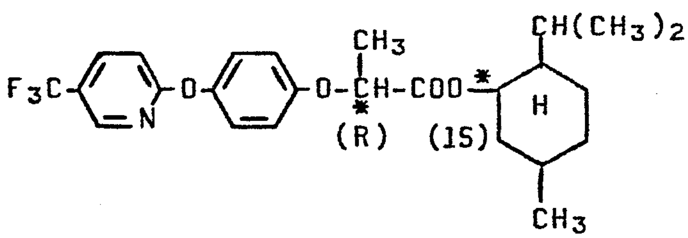

(I-6)

Ausbeute : 92,5 % der Theorie

$n_D^{22}$ = 1,4992

Drehwert : $[\alpha]_D^{24}$ = +29,4°

(1-molare Lösung in Chloroform; Küvettenlänge 10 cm)

Beispiel 7 :

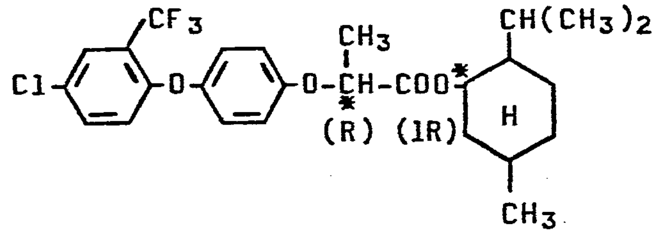

(I-7)

Ausbeute : 96 % der Theorie

$n_D^{22}$ = 1,5105

Drehwert : $[\alpha]_D^{24}$ = -4,1°

(1-molare Lösung in Chloroform; Küvettenlänge 10 cm)

Beispiel 8 :

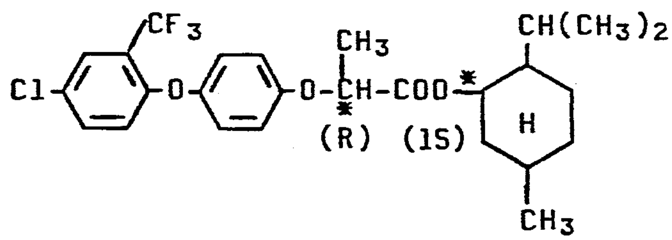

(I-8)

Ausbeute : 82,5 % der Theorie

$n_D^{22}$ = 1,5101

Drehwert : $[\alpha]_D^{24}$ = +20,1°

(1-molare Lösung in Chloroform; Küvettenlänge 10 cm)

Beispiel 9 :

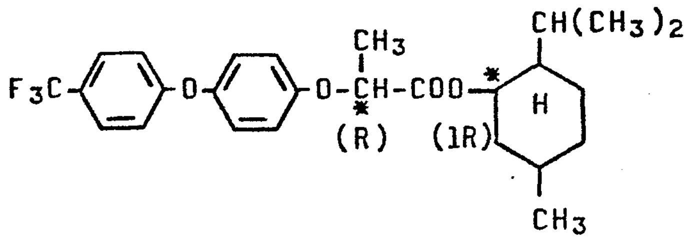

(I-9)

Ausbeute : 87 % der Theorie

$n_D^{22}$ = 1,5026

Drehwert : $[\alpha]_D^{24}$ = -3,5°

(1-molare Lösung in Chloroform; Küvettenlänge 10 cm)

Beispiel 10 :

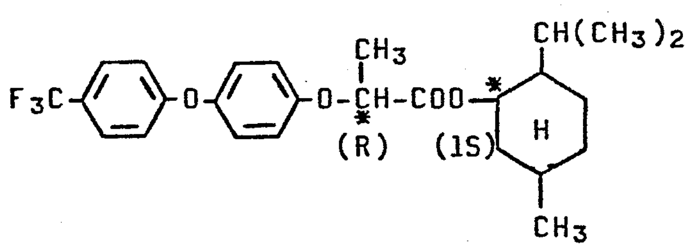

(I-10)

Ausbeute : 94 % der Theorie

$n_D^{22}$ = 1,5012

Drehwert : $[\alpha]_D^{24}$ = +25,6°

(1-molare Lösung in Chlorofom; Küvettenlänge 10 cm)

Beispiel 11 :

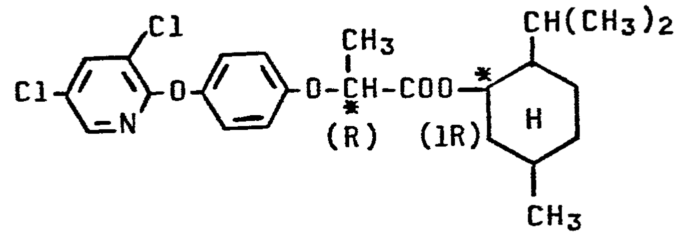

(I-11)

Ausbeute : 64 % der Theorie

Beispiel 12 :

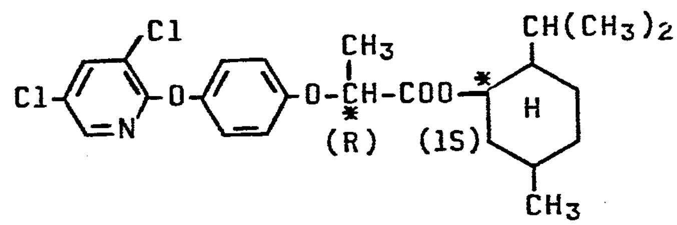

(I-12)

Ausbeute : 75 % der Theorie

Drehwert : $[\alpha]_D^{24} = -0{,}1°$

(1-molare Lösung in Chloroform; Küvettenlänge 10 cm)

Beispiel 13 :

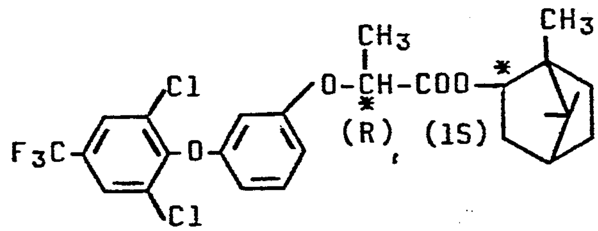

(I-13)

Ausbeute : 92 % der Theorie

$n_D^{21} = 1{,}5257$

Drehwert : $[\alpha]_D^{24} = -5°$

(1-molare Lösung in Chloroform; Küvettenlänge 10 cm)

Beispiel 14 :

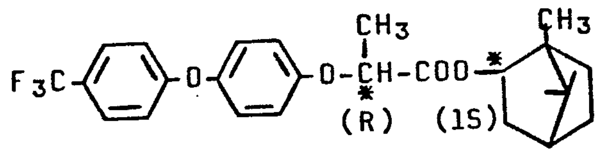

(I-14)

Ausbeute : 96 % der Theorie

$n_D^{21} = 1{,}5148$

Drehwert : $[\alpha]_D^{24} = +2{,}91°$

(1-molare Lösung in Chloroform; Küvettenlänge 10 cm)

Beispiel 15 :

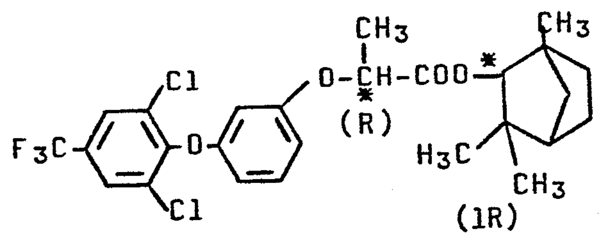

(I-15)

Ausbeute : 95 % der Theorie

$n_D^{21} = 1{,}5243$

Drehwert : $[\alpha]_D^{24} = +11{,}1°$

(1-molare Lösung in Chloroform;Küvettenlänge 10 cm)

Beispiel 16 :

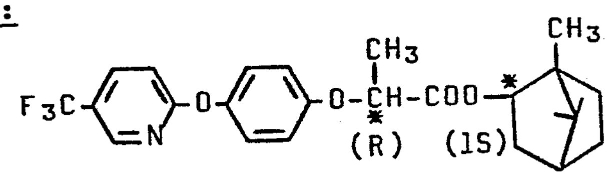

(I-16)

Ausbeute : 88 % der Theorie

$n_D^{21} = 1{,}5135$

Drehwert : $[\alpha]_D^{24} = +7{,}4°$

(1-molare Lösung in Chloroform, Küvettenlänge 10 cm)

Beispiel 17 :

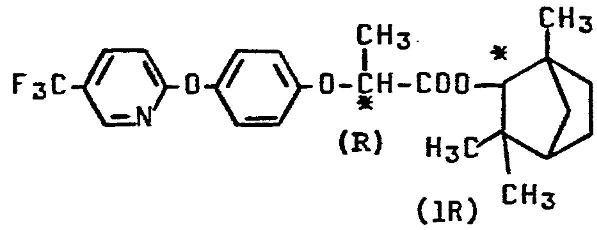

(I-17)

Ausbeute : 90 % der Theorie

$n_D^{21}$ = 1,5140

Drehwert : $[\alpha]_D^{24}$ = +22,2°

(1-molare Lösung in Chloroform; Küvettenlänge 10 cm)

Beispiel 18 :

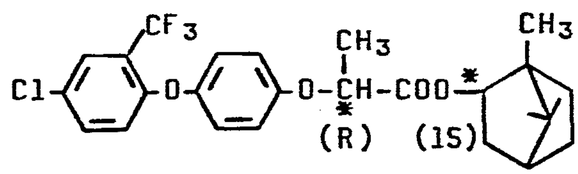

(I-18)

Ausbeute : 90 % der Theorie

$n_D^{21}$ = 1,5236

Drehwert : $[\alpha]_D^{24}$ = + 0,9°

(1-molare Lösung in Chloroform; Küvettenlänge 10 cm)

Beispiel 19 :

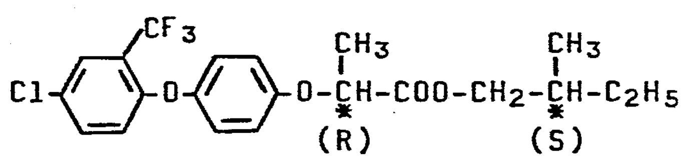

(I-19)

Ausbeute : 95 % der Theorie

$n_D^{21}$ = 1,5093

Drehwert : $[\alpha]_D^{24}$ = + 7,5°

(1-molare Lösung in Chloroform;Küvettenlänge 10 cm)

Beispiel 20 :

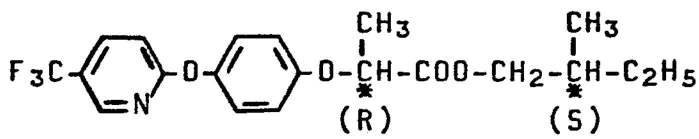
(I-20)

Ausbeute : 96 % der Theorie

$n_D^{21} = 1,5010$

Drehwert : $[\alpha]_D^{24} = + 12,5°$

(1-molare Lösung in Chloroform; Küvettenlänge 10 cm)

Ausgangsstoffe der Formel (V) :

Beispiel (21):

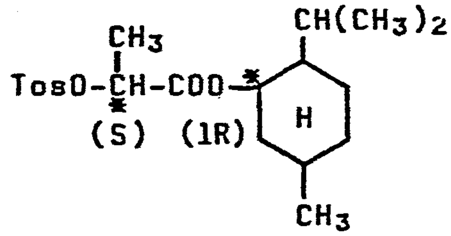

(V-1)

( Tos = 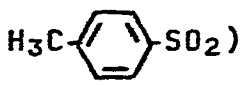 )

Ein Gemisch von 26,2 g (0,1 Mol) des S-Enantiomeren von Milchsäurechloridtosylat und 15 g (0,1 Mol) (1R,3R,4S)-Menthol wurde 4 Stunden auf 130°C erhitzt und anschließend auf 20°C abgekühlt.

Man erhielt 32,6 g (82 % der Theorie) 2-Tosyloxy-(2S)-propionsäure-(1R,3R,4S)-menth-3-ylester.

Brechungsindex : $n_D^{22}$ = 1,4982

Drehwert : $[\alpha]_D^{24}$ = -24,5°

(1-molare Lösung in Chloroform; Küvettenlänge 10 cm)

Analog wurden die übrigen Verbindungen der Formel (V) hergestellt :

<u>Beispiel (22):</u>

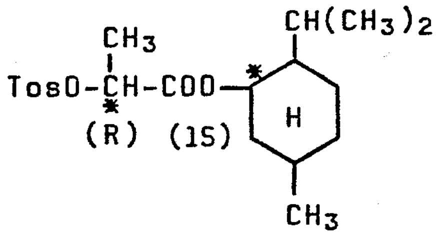

(V-2)

Ausbeute : 88 % der Theorie

$n_D^{22}$ = 1,4959

Drehwert : $[\alpha]_D^{24}$ = -2,3°

(1-molare Lösung in Chloroform; Küvettenlänge 10 cm)

0151429

In dem nachstehend beschriebenen biologischen Test wurde die folgende Verbindung als Vergleichssubstanz eingesetzt :

(A) = 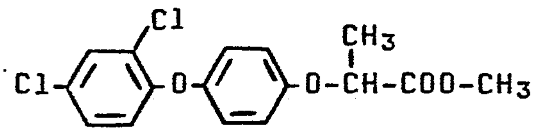

2-[4-(2,4-Dichlorphenoxy)-phenoxy]-propionsäuremethylester

(bekannt aus DE-OS 22 23 894).

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

In diesem Test zeigte der erfindungsgemäße Wirkstoff (I-20) eine bessere selektive herbizide Wirksamkeit als die Vergleichssubstanz (A).

Patentansprüche

1. R-Enantiomere von Phenoxypropionsäure-Derivaten der Formel

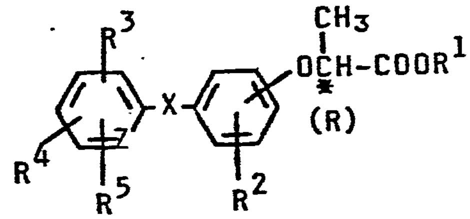

(I)

in welcher

Z für Stickstoff oder die Gruppierung -CR steht, worin
R für Wasserstoff oder Halogen steht,

X für Sauerstoff oder Schwefel steht,

$R^1$ für Alkyl, gegebenenfalls substituiertes Cycloalkyl, gegebenenfalls substituiertes Cycloalkenyl, gegebenenfalls substituiertes Bicycloalkyl oder gegebenenfalls substituiertes Bicycloalkenyl mit jeweils mindestens einem asymmetrisch substituierten Kohlenstoffatom steht,

$R^2$ für Wasserstoff, Cyano oder Nitro steht,

$R^3$ und $R^4$ gleich oder verschieden sind und für Wasserstoff oder Halogen stehen und

$R^5$ für Wasserstoff, Halogen oder Trihalogenmethyl steht.

2. R-Enantiomere von Phenoxypropionsäure-Derivaten der Formel (I), in denen

Z für Stickstoff oder die Gruppierung -CR steht, worin

R für Wasserstoff, Chlor oder Brom steht,

X für Sauerstoff oder Schwefel steht,

$R^1$ für Alkyl mit 4 bis 12 Kohlenstoffatomen mit mindestens einem asymmetrisch substituierten Kohlenstoffatom sowie für (1R,2S)-Fenchyl, (1S,2R)-Bornyl, (1R,2R)-iso-Bornyl, (1R)-Myrtenyl, (1S,2S,4S)-Dihydrocarvyl, (1R,3R,4S)-Menthyl oder (1S,3S,4R)-Menthyl steht,

$R^2$ für Wasserstoff, Cyano oder Nitro steht,

$R^3$ für Wasserstoff, Chlor oder Brom steht,

$R^4$ für Wasserstoff, Chlor oder Brom steht und

$R^5$ für Wasserstoff, Fluor, Chlor, Brom, Trifluormethyl, Difluorchlormethyl oder Trichlormethyl steht.

3. Verfahren zur Herstellung von R-Enantiomeren von Phenoxypropionsäure-Derivaten der Formel

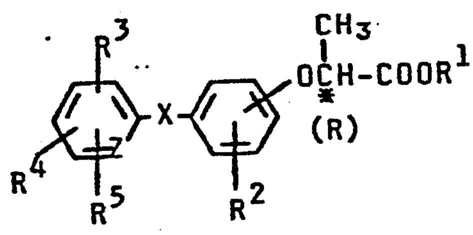

(I)

in welcher

Z für Stickstoff oder die Gruppierung -CR steht,

worin
R für Wasserstoff oder Halogen steht,

X für Sauerstoff oder Schwefel steht,

$R^1$ für Alkyl, gegebenenfalls substituiertes Cycloalkyl, gegebenenfalls substituiertes Cycloalkenyl, gegebenenfalls substituiertes Bicycloalkyl oder gegebenenfalls substituiertes Bicycloalkenyl mit jeweils mindestens einem asymmetrisch substituierten Kohlenstoffatom steht,

$R^2$ für Wasserstoff, Cyano oder Nitro steht,

$R^3$ und $R^4$ gleich oder verschieden sind und für Wasserstoff oder Halogen stehen und

$R^5$ für Wasserstoff, Halogen oder Trihalogenmethyl steht,

dadurch gekennzeichnet, daß man

(a) R-Enantiomere von Phenoxypropionsäurechloriden der Formel (II),

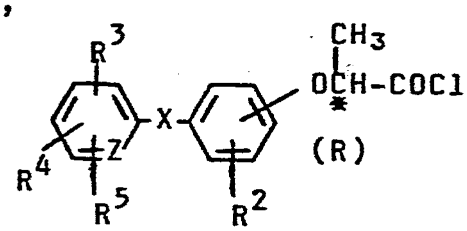

(II)

in welcher

$R^2$, $R^3$, $R^4$, $R^5$, Z und X die oben angegebenen Bedeutungen haben,

mit Verbindungen der Formel (III),

$$R^1OH \qquad (III)$$

in welcher

$R^1$ die oben angegebene Bedeutung hat,

gegebenenfalls in Gegenwart eines Säureakzeptors sowie gegebenenfalls in Gegenwart eines Verdünnungsmittels umsetzt, oder

(b) Phenole der Formel (IV),

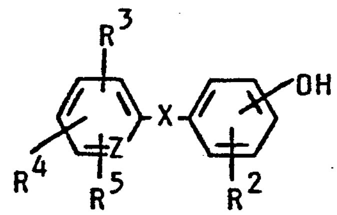

(IV)

in welcher

$R^2$,$R^3$,$R^4$,$R^5$, Z und X die oben angegebenen Bedeutungen haben,

mit S-Enantiomeren von Propionsäure-Derivate der Formel (V),

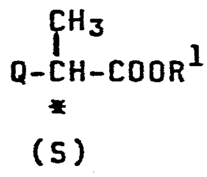

(V)

in welcher

$R^1$ die oben angegebene Bedeutung hat und

Q für Tosylat oder Mesylat steht,

gegebenenfalls in Gegenwart eines Säureakzeptors sowie gegebenenfalls in Gegenwart eines Verdünnungsmittels umsetzt,

oder

(c) R-Enantiomere von Phenoxypropionsäure-Derivaten der Formel (Ia),

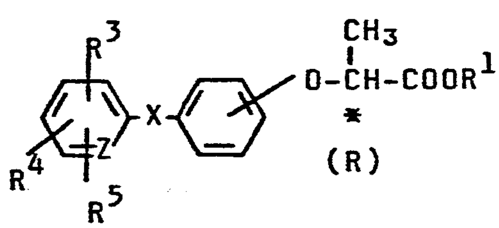

(Ia)

in welcher

$R^1$,$R^3$,$R^4$,$R^5$, Z und X die oben angegebenen Bedeutungen haben,

mit Kupfernitrat in Gegenwart von Essigsäureanhydrid und Eisessig umsetzt.

4. Herbizide Mittel, gekennzeichnet durch einen Gehalt an mindestens einem R-Enantiomeren eines Phenoxypropionsäure-Derivates der Formel (I).

5. Verfahren zur Bekämpfung von Unkräutern, dadurch gekennzeichnet, daß man R-Enantiomere von Phenoxypropionsäure-Derivaten der Formel (I) auf die Unkräuter und/oder deren Lebensraum ausbringt.

6. Verwendung von R-Enantiomeren von Phenoxypropionsäure-Derivaten der Formel (I) zur Bekämpfung von Unkräutern.

7. Verfahren zur Herstellung von herbiziden Mitteln, dadurch gekennzeichnet, daß man R-Enantiomere von Phenoxypropionsäure-Derivaten der Formel (I) mit Streckmitteln und/oder oberflächenaktiven Stoffen vermischt.

8. S-Enantiomere von Propionsäure-Derivaten der Formel

$$Q-\underset{\underset{(S)}{*}}{\overset{\overset{CH_3}{|}}{C}H}-COOR^1 \qquad (V)$$

in welcher

$R^1$ für Alkyl, gegebenenfalls substituiertes Cycloalkyl, gegebenenfalls substituiertes Cycloalkenyl, gegebenenfalls substituiertes Bicycloalkyl oder gegebenenfalls substituiertes Bicycloalkenyl

mit jeweils mindestens einem asymmetrisch substituierten Kohlenstoffatom steht und

Q für Tosylat oder Mesylat steht.

9. Verfahren zur Herstellung von S-Enantiomeren von Propionsäure-Derivaten der Formel

$$Q-\underset{*}{\overset{CH_3}{\overset{|}{C}}}H-COOR^1 \qquad (V)$$

(S)

in welcher

$R^1$ für Alkyl, gegebenenfalls substituiertes Cycloalkyl, gegebenenfalls substituiertes Cycloalkenyl, gegebenenfalls substituiertes Bicycloalkyl oder gegebenenfalls substituiertes Bicycloalkenyl mit jeweils mindestens einem asymmetrisch substituierten Kohlenstoffatom steht und

Q für Tosylat oder Mesylat steht,

dadurch gekennzeichnet, daß man S-Enantiomere von Propionylchloriden der Formel

$$Q-\underset{*}{\overset{CH_3}{\overset{|}{C}}}H-CO-Cl \qquad (VI)$$

(S)

in welcher

Q die oben angegebene Bedeutung hat,

mit Verbindungen der Formel (III),

$$R^1OH \qquad (III)$$

in welcher
$R^1$ die oben angegebene Bedeutung hat,

gegebenenfalls in Gegenwart eines Säureakzeptors sowie gegebenenfalls in Gegenwart eines Verdünnungsmittels umsetzt.

10. R-Enantiomeres des Phenoxypropionsäure-Derivates oder Formel